# EUROPEAN PATENT APPLICATION

(11) **EP 3 263 000 A1**
(43) Date of publication of application: **03.01.2018**
(21) Application number: 17178526.4
(22) Date of filing: 28.06.2017
(51) Int. Cl.: A47K 5/12, G01F 23/72, G06F 19/00

(54) **SOAP DISPENSER AND DATA COMMUNICATION NETWORK SYSTEM INCLUDING SAME**

(30) Priority: 28.06.2016 NL 2017056
(71) Applicant: Vendor B.V., 5047 TT Tilburg (NL)
(72) Inventor: Baijens, Martijn Cornelis Adriaan, 5047 TT Tilburg (NL)
(74) Representative: V.O.

(57) **Abstract**

Soap dispenser (1) that includes a housing with a support base (3) for mounting to a wall surface. A cartridge (37) containing a supply of soap (35) is removably supported on the support base (3), and has a liquid outlet (39). The soap dispenser (1) further includes a buffer reservoir (33) having a liquid inlet (40) for detachably receiving the liquid outlet (39) of the cartridge (37) for establishing fluid communication with the buffer reservoir (33), and a dosing pump (31) attached the support base in fluid communication with the buffer reservoir (33). The dosing pump (31) is arranged for selective actuation by a user, and has a dispensing outlet (5) for expelling soap. An indicator rotor (15), which is associated with the housing is positioned outside of the soap content (35) and is coupled by a magnetic field to a float (41) within the buffer reservoir.

## Description

The invention relates to an indicator on a soap dispenser, which shows when a cartridge needs to be refilled or replaced by a cleaning service staff, or the like.

Where in this specification reference is made to "soap" this expression is intended to encompass any liquid or viscous detergents, as well as any liquid or viscous disinfectants that are suitable for being dispensed from a dispensing device that will usually be wall mounted in proximity of a water tap.

Soap dispensers for dosing a viscous substance from an exchangeable refill container are generally known from applicants European paten publication EP1098584. While these known devices have been satisfactory for their intended purpose, it has been noticed that the means available to detect the need to replace the refill container have at times lead to confusing interpretations. Additionally surveillance of such known soap dispensers has only been possible on location and thus only by a few individuals having physical access to the location of the relevant soap dispenser.

Accordingly it is an object of the present invention to propose an improved soap dispenser and method for its use in a data communication network. In a more general sense it is thus an object of the invention to overcome or reduce at least one of the disadvantages of the prior art. It is also an object of the present invention to provide alternative solutions which are less cumbersome in execution and operation and which moreover can be made relatively inexpensively. Alternatively it is an object of the invention to at least provide a useful alternative.

To this end the invention generally provides for a soap dispenser and a system using it in a data communication network as defined by one or more of the appended claims. More in particular the invention provides a soap dispenser, which includes: a housing with a support base for mounting to a wall surface; a cartridge containing a supply of soap removably supported on the support base, and having a liquid outlet; a buffer reservoir having a liquid inlet for detachably receiving the liquid outlet of the cartridge for establishing fluid communication with the buffer reservoir; and a dosing pump attached the support base in fluid communication with the buffer reservoir, and arranged for selective actuation by a user, the dosing pump further having a dispensing outlet for expelling soap, wherein an indicator rotor associated with the housing is positioned outside of the soap content and is coupled by a magnetic field to a float within the buffer reservoir. Advantageously the indicator rotor can be journaled at its upper and lower axial ends for rotation about a substantially vertical axis, and accommodates an indicator permanent magnet. In this regard the float can be mechanically guided for vertical translating movement, and carries a float permanent magnet. The float magnet can also optionally be positioned on top of the float to be at any time positioned in an air space above an upper soap level in the buffer reservoir.

Primary advantages of such an arrangement are that a signal on a time that a refill bottle or cartridge is empty can be made unambiguously "binary" in character, and clearly distinguishes between empty and not-empty. By positioning the float magnet freely in the air space above soap, contamination and crystallization of soap will have no effect on its operation.

Advantageously the float magnet in the soap dispenser can have its north pole positioned above its south pole to be aligned in a substantially vertical plane, and the indicator magnet can have its north and south poles aligned in a substantially horizontal plane. In this regard the float magnet with its permanent magnetic field will interferes with the permanent magnetic field the indicator magnet of the indicator rotor, so that vertical movement of the float magnet will change polarity of the magnetic field to which the indicator magnet is exposed. This will result in a mechanism that changes polarity at the time that the bottle or cartridge is empty. By rotating the indicator rotor 180° about its vertical axis. The reversing of polarity is effected by the float magnet that vertically changes polarity from North to South. This float magnet drops together with the float and with the lowering fluid level in the buffer reservoir at the time that the bottle or cartridge is empty. By positioning the polarity change of the magnetic field within a distance of about 4 to 8 mm of float travel, the exact point in which reversing of polarity happens can be positioned as close as possible to a point that the fluid actually starts lowering in the buffer reservoir. Thereby the indicator, which is placed outside of the liquid, will turn instantly through 180°. The cleaning staff will thus clearly notice that the bottle or cartridge is empty and needs replacement. For a still clearer alert to the cleaning staff the indicator rotor can be vertically divided into contrasting colored opposite halves.

Optionally the soap dispenser of the invention can further include a printed circuit board (PCB), and associated therewith a local area network (LAN) module for transmitting data and/or receiving data within a network. Further associated therewith the PCB can include a reed switch, which in turn is associated with the indicator rotor to open and close a contact under influence of the polarity of the magnetic field. It is also an option for PCB to include sensors for recognizing a cartridge identity. With such a structure also a link can be made between the indicator rotor and the reed switch on a printed circuit board (PCB). In an energy-efficient and unambiguous way it will then be signaled when the bottle or cartridge is empty. A thus obtained digital readout is then also transmitted over a wireless network (e.g. Bluetooth, LORA), and carried through to a remote server. The status is then also available to be checked on a smart device (tablet, smartphone, smartwatch). As a secondary advantage also the number of dosing draws from the soap dispenser may be monitored by sensor components on the printed circuit board (PCB).

The invention also relates to a network system for remotely providing service functionalities for a soap dispenser, comprising at least one cartridge based soap dispenser as explained above, and a remote server in data communication with this at least one soap dispenser via a communication network, the at least one soap dispenser then being adapted to monitor dispenser related parameters, and to submit the monitored parameters to the remote server, and wherein the remote server is adapted to initiate a service functionality depending on the submitted parameters.

The so obtained smart indication can be applied to a liquid dispenser, such as for soaps, lotions, or disinfection gels. In the dispenser after it has been filled with a supply bottle or a cartridge, the liquid contents runs into a buffer reservoir from which the fluid is being pumped. When the cartridge filling is empty, there will still be a back-up supply in the buffer reservoir to cover an interval up to the moment of cartridge replacement. In this way, the liquid content can be completely emptied without there being any intermediate absence of supply to the end user. The buffer reservoir accommodates the mechanically controlled float carrying float magnet. The float magnet by its magnetic field acts on indicator magnet located in the indicator rotor which is positioned outside of the fluid containing bottle or cartridge. The indicator rotor can thereby have a white color on one side and be red or orange colored on its other side. When the liquid level drops, the float with the float magnet descents and the magnetic field changes polarity with respect to the magnetic field of the rotor. The indicator rotor as a result will turn instantly and thereby indicate that the bottle or cartridge must be exchanged. At this point in time the fluid in the buffer reservoir is decreasing, and hence the bottle or cartridge is already empty and can be replaced, without spilling. In this way it will become unambiguously clear for the cleaning staff or other qualified persons in surveillance of the equipment what they are required to do. Hence the typical prior art analog signal that would be provided by a level gauge is now replaced by an objective binary indication that only distinguishes between empty, and not-empty.

Also variants are conceivable in which the indicator rotor has multiple positions. For example, indicated by colors white, orange, red for content levels of respectively 100%, 30%, or less than 10%. This would be achieved by placing multiple magnets on top of each other in the indicator rotor each under a different angle. If the float with its float magnet moves along the indicator rotor, this will always assume the relevant angular position. In this way it will be possible to expand the indicator function with multiple modes. For example half empty, completely empty (by using differentiating colors such as white, orange, and red).

The invention also relates to a system for remotely providing service functionalities for a soap dispenser, comprising at least one cartridge based soap dispenser, having at least one sensor for monitoring dispenser related parameters, a communication module for enabling communication with a remote server via a communication network and for submitting the monitored parameters to the remote server, and wherein the communication module is adapted to receive from the server an indication of a necessary service functionality depending on the submitted parameters. Optionally such a soap dispenser system can comprise a user interface for outputting the received indication to a user. Also or alternatively the communication module in the system can be a bidirectional interface.

Optionally the system can also comprise a data storage for storing the monitored parameters, and the communication module can be arranged to submit the stored parameters upon a request received from the server.

Optionally the system can further comprise a control unit that is arranged for starting a maintenance function if an indication of a corresponding service functionality is received by the communication module.

The system of the invention can also comprise a soap dispenser having a control module, with the communication module connected to the control module, a communication program for communication to the server can then be carried out by the control module of the soap dispenser or by a control module included in the communication module.

The communication network of the system can additionally comprise a dedicated network between the communication module and a public network. The dedicated network can then be a wireless network.

The invention also provides for a system for remotely providing service functionalities for a soap dispenser, which system comprises a cartridge based soap dispenser being adapted for dispensing a liquid soap product by receiving a cartridge in a cartridge receiving base part, and a remote server that is in data communication with the soap dispenser via a communication network, the soap dispenser in particular being adapted to monitor dispenser related parameters and to submit these monitored parameters to the server, while the server is adapted to initiate a service functionality depending on the submitted parameters.

The invention further provides for a soap dispenser system that comprises a control unit and a communication module designed to support a method of initiating a service functionality selected from the group consisting of: offering a service to the customer; taking steps for automatically providing a service to the customer; indicating to the customer an upcoming maintenance period; indicating to the customer shortage of a supply at the dispenser needed for dispensing a liquid soap product; and indicating to the customer the need for replacement of a part of the dispenser.

The invention also encompasses a method for remotely providing service functionalities for at least one cartridge based soap dispenser, the at least one cartridge based soap dispenser is adapted for dispensing a liquid soap product by receiving a cartridge in a cartridge receiving base part, and is in data communication with a remote server via a communication network, the method in particular comprises the steps of: monitoring parameters related to the dispenser, submitting the monitored parameters to the server and initiating through the server a service functionality depending on the submitted parameters. In this method the step of initiating a service functionality can comprise at least one step selected from the group consisting of: offering a service to the customer; taking steps for automatically providing a service to the customer; indicating to the customer an upcoming maintenance period; indicating to the customer a shortage of a supply within the dispenser needed for dispensing a liquid soap product; and indicating to the customer the need to replace a part of the dispenser. Optionally the taking of steps for automatically providing a service to the customer comprises at least one step selected from the group consisting of: arranging an appointment with a maintenance assistant; setting up a communication with a help desks; and dispensing shipment of supplies and/or spare parts for the dispenser. Alternatively or additionally the step of monitoring can comprise monitoring by the at least one soap dispenser of the number of consumed cartridges; and automatically recognizing by the at least one liquid soap product dispenser the type of each consumed refill cartridge, and wherein the step of initiating a service functionality can comprise comparing for each type of refill cartridge an initial amount of refill cartridges at the soap dispenser with the actual remaining amount of refill cartridges, and in case of detecting a reaching of a shortage of refill cartridges at the soap dispenser, generating automatically an instruction: to invite the customer to make an order for a shipment of a new stock of refill cartridges, or to prepare a shipment of a new stock of refill cartridges for supply to said customer.

Optionally the method can also comprise the steps of: accepting by the soap dispenser a user identification indicating user settings regarding liquid soap product dosing; adapting the liquid soap product dosing process according to the user settings; and submitting information on the accepted user identification to the server.

For this purpose the dispenser has a printed circuit board (PCB) with a wireless local area network module (LAN module), and also a variety of sensors and reed contact switches. The printed circuit board (PCB) thereby can communicate the status and signals of sensors to a network. The following parameters of the dispenser can thereby be read: (i) Position of the indicator rotor and thus the possibility for the message to refill; (ii) Position of the dosing pump setting (Economy-Medium-Full); (iii) Number of pump or dosages strokes and the possibility to acquire insight for hand hygiene and general use; and/or (iv) Article number on item label through binary code and optical sensor.

Further advantageous aspects of the invention will become clear from the appended description and in reference to the accompanying drawings, in which:
Figure 1 is a general exterior view of a liquid soap dispenser in accordance with the invention;
Figure 2 is a fragmentary cross section through the soap dispenser of Figure 1 showing a still partly filled cartridge containing liquid soap;
Figure 3 is a fragmentary cross section similar to Figure 2, but showing an empty liquid soap cartridge; and
Figure 4 is a schematic representation of several soap dispensers within a data communication network.

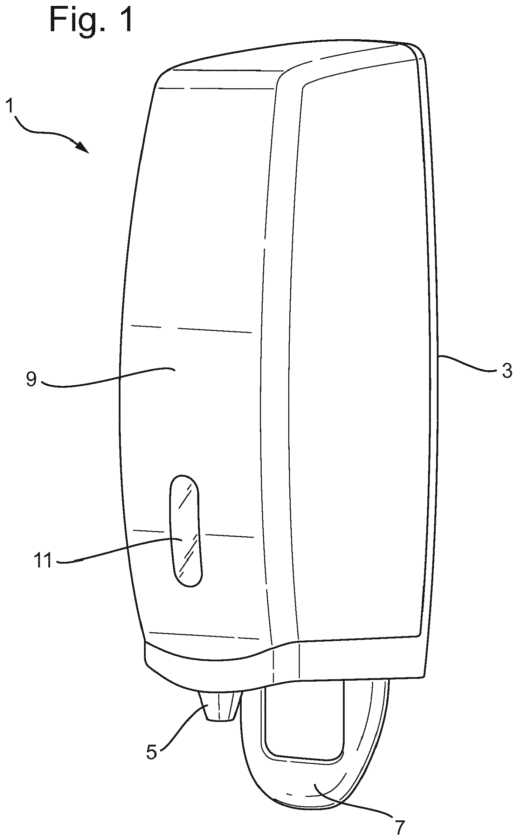

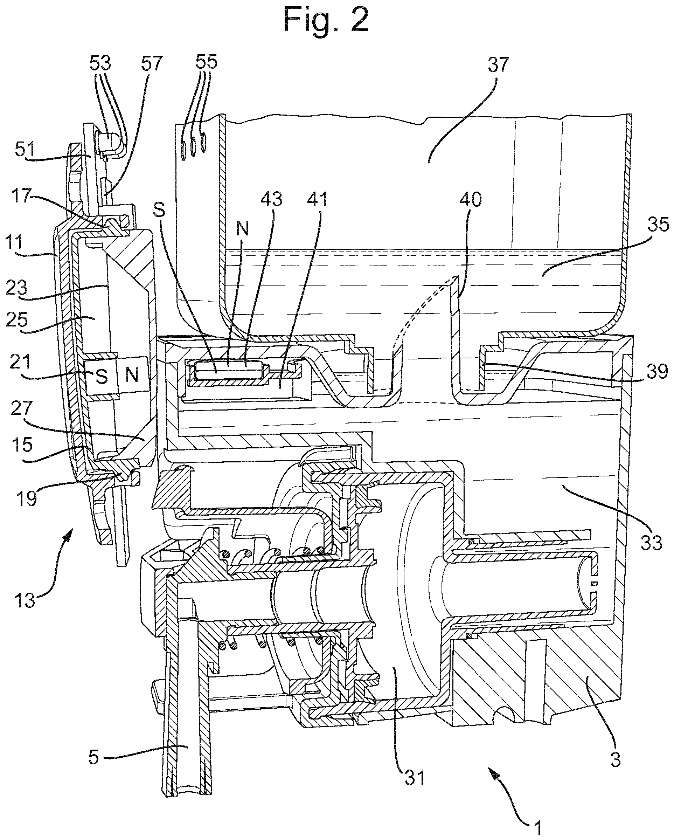

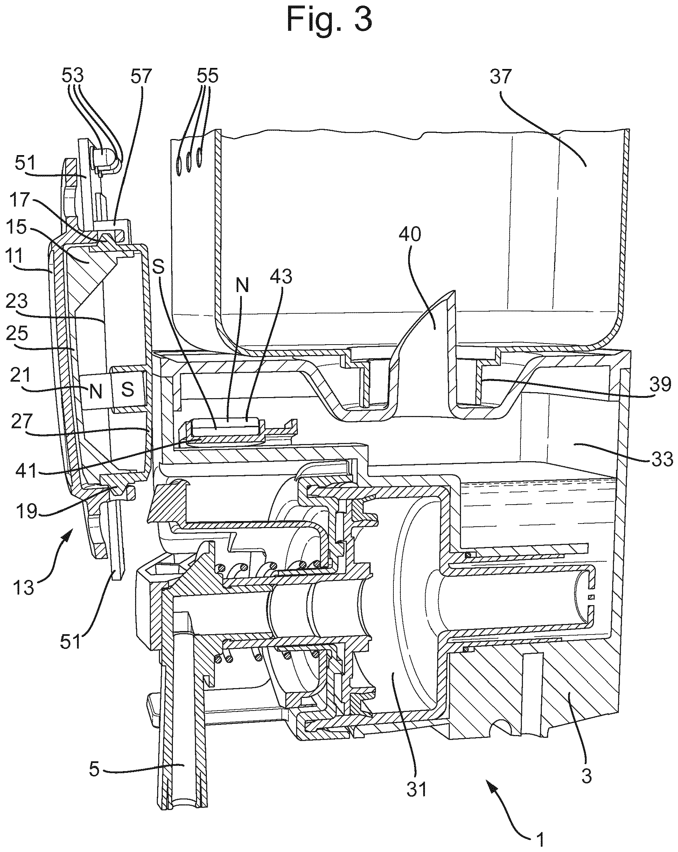

As shown in Figure 1 a liquid soap dispenser 1 has a wall mount base part 3. At a lower end of the wall mount base part 3 is arranged a discharge spout 5, and an operating handle 7 for operating a dosing pump (reference numeral 31 in Figures 2 and 3). Upon operation of the operating handle 7 a predetermined amount of liquid soap will be expelled from the discharge spout 5. The soap that is to be dispensed from the discharge spout 5 is contained, as is conventional, within a cartridge or bottle (reference numeral 37 in Figures 2 and 3) located on the wall mount base 3, and in use is covered by a removable outer housing or lid 9. The liquid soap cartridge can be formed in many ways, as a bottle, as a flexible pack, as a bag in box, or as a rigid container or the like. For the invention it is not at all important to which form of cartridge the soap dispenser 1 is adapted. The removable outer housing, or lid 9 is provided with a window 11, which allows a filling indicator to be viewed there through.

As will now be seen in Figure 2 the window 11 is part of an indicator sub assembly 13, which includes an indication rotor 15. The indicator rotor 15 is journaled at its upper and lower ends 17, 19 for rotation about a vertical axis. Within the indicator rotor 15 is accommodated a permanent indicator magnet 21. The indicator rotor 15 is split along a vertical division 23 into first and second housing halves 25, 27. The first housing half 25 is associated with the south pole of the indicator magnet 21, and can have a first color, such as white. The second housing half 27 is associated with the north pole of the indication magnet 21, and can have a second color, such as red.

As further shown in Figure 2, the discharge spout 5 is associated with a dosing pump 31, which can be operated by the operating handle 7 (only shown in Figure 1) in a manner which is conventional and does not need further description in the context of the present invention. Formed within the wall mount base 3 is a buffer reservoir 33, from which the dosing pump 31 is fed with liquid soap 35. The liquid soap 35 as shown in Figure 2 is still present in the bottom part of the exchangeable soap cartridge 37. The exchangeable soap cartridge has a liquid outlet 39, by which it is in fluid communication with an inlet 40 of the buffer reservoir 33. Accommodated within the buffer reservoir 33 is also a float 41 having a float magnet 43. The float magnet 43 differs from the indicator magnet 21, in that it has its north pole positioned above its south pole in a common substantially vertical plane. The indicator magnet 21 in contrast has its north and south poles arranged in a common substantially horizontal plane. As long as the float 41 is in its upper most position, with a sufficient supply of liquid soap 35 to completely fill the buffer reservoir 33, the south pole of the float magnet 43 will be positioned to attract the north pole of the indicator magnet 21. When the float 41 drops, as is shown in Figure 3 for an empty buffer reservoir 33, the indicator magnet 21 has its south pole attracted by the upper north pole of the float magnet 43.

It is further seen in Figures 2 and 3 that the indicator sub assembly 13 is also associated with a printed circuit board (PCB) 51 that extends alongside the indicator rotor 15. The printed circuit board 51 in this example has amongst others a wireless local area network module (LAN module), as well as a variety of sensors and reed contacts. In particular the printed circuit board 15 as shown in Figures 2 and 3 has triple optical sensors 53 for detection of triple coding dots 55 on the exterior surface of the cartridge 37. These coding dots 55 can have individually different colors or shades to enable recognition by the sensors 53 of cartridges 37 differing in contents or article number. The number of individual combinations allowed by triple sensors and triple dots is sufficient for the present example, but clearly additional dots and sensors may be added to allow a greater number of variations.

The thus obtained smart form of indication can be applied to liquid dispenser such as for soaps, lotions, and/or disinfection gel within the definition of "soap" as used throughout the present description.

The dispenser 1 can be filled with a bottle or a cartridge 37. The liquid 35 runs into a buffer reservoir 33 from which the fluid is being pumped. When the cartridge filling is empty, there still is a back-up supply in the buffer reservoir 33 to cover an interval up to the moment of cartridge replacement. In this way, the liquid content 35 can be completely emptied without there occurring any absence of supply to the end user. In the buffer reservoir 33 is located the mechanically guided float 41 with the float magnet 43. The float magnet 43 by its magnetic field attracts the indicator magnet 21 located in the indicator rotor 15, which is positioned outside of the fluid containing bottle or cartridge 37 and buffer reservoir 33. The indicator rotor 15 can have a white color on one side and be red or orange colored on its other opposite side.

When the liquid level drops, the float 41 with the float magnet 43 descents and the magnetic field changes polarity with respect to the magnetic field of the indicator rotor 15 with the indicator magnet 21. The indicator rotor 15 as a result will turn instantly and thereby indicate that the bottle or cartridge 33 needs to be exchanged. At this point in time the fluid 35 in the buffer reservoir 33 is decreasing in volume, and hence the bottle or cartridge 37 is already empty.

In this way it will become unambiguously clear for the attending cleaning staff or other qualified persons in surveillance of the equipment what is required to be done. The typical analog signal that would be provided by a conventional level gauge is now replaced by an indisputable binary indication that only distinguishes between empty, and not-empty.

By means of the LAN module the printed circuit board 51 can communicate the cartridge refill, recognized by the triple optical sensor 53 to a network, as will be further explained below.

Also positioned on the printed circuit board 51, in the vicinity of the indicator rotor 15 is a reed switch 57 that is appropriately positioned to be operated by the indicator magnet 21 to open or close an electrical contact. The relevant full or empty signal generated via the reed switch 57 by the position of the indicator rotor 15 can also be communicated to a network by the LAN module of the printed circuit board 51.

Additional parameters of the dispenser 1 that can be detected by components of the printed circuit board 51, include settings for the dosing pump 31, and/or the number of pump actuations. Such additional parameters can also be communicated to a network.

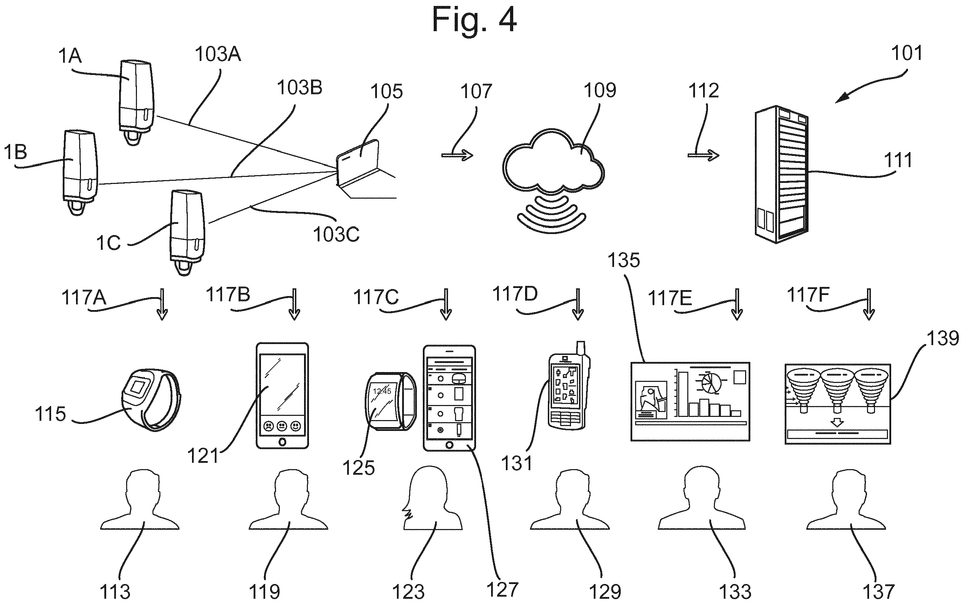

As schematically shown in Figure 4, a plurality of soap dispensers 1A, 1B, 1C present at a single client location can be part of a general communication network 101. The soap dispensers 1A, 1B, 1C of the single client location can each have a dedicated data communication channel 103A, 103B, 103C to a telemetry device 105, which can be a communication module. A local server, or a modem. The telemetry device 105 uses a first data communication channel 107 with the internet 109. Via the internet 109 communication is available with a remote server 111 through a second data communication channel 112. Users, attendants and suppliers associated with the soap dispensers 1,1A-1C can have differentiated access via the network 101 to date exchanged between the telemetry device 105 and the remote server 111. For such access the users, attendants, and suppliers can each use an appropriate user device having network capabilities. In the example illustrated in Figure 4 a professional end user 113 may use a smart watch 115 to obtain information about hygiene monitoring and hygiene compliance through communication channel 117A. A public end user 119 in this example may perform user interaction, obtain hygiene advice or participate in a loyalty program using a mobile or smart phone 121 as the user device having network capabilities. The necessary data will be transmitted to and from the internet 109 via a communication channel 117B. A member of a cleaning staff 123 may have access to information relating to a refill moment or product advice through a smart watch 125, or a mobile phone 127 using communication channel 117C. On the supplier side a member of the service staff 129 may be provided with a personal digital assistant (PDA) 131, to obtain access to or perform service planning, and may also perform service optimization via communication channel 117D. Also at the supplier side a facility manager 13, and/or a sales person 137 may each have access to user and customer statistics via personal computers 135, 139 using communication channel 117E, 117F respectively.

Thus the soap dispenser 1, 1A-1C has a printed circuit board (PCB) 51 with a wireless local area network module (LAN module), and also a variety of sensors 53 and one or more reed contact switches, such as 57. The printed circuit board (PCB) 51 can communicate the status and signals of sensors within a network 101.

The following parameters of the dispenser can be read:
- Position of the indicator rotor by reed switch 57, and thus offering the possibility for a message to refill;
- Position of a dosing pump setting (such as: Economy-Medium-Full);
- Number of pump or dosages strokes and the possibility to acquire insight for hand hygiene and general use; and/or
- Article number on a cartridge item label through coding dots 55, proving a binary code, and optical sensors 53.

Accordingly there is described a soap dispenser 1 that includes a housing with a support base 3 for mounting to a wall surface. A cartridge 37 containing a supply of soap 35 is removably supported on the support base 3, and has a liquid outlet 39. The soap dispenser 1 further includes a buffer reservoir 33 having a liquid inlet 40 for detachably receiving the liquid outlet 39 of the cartridge 37 for establishing fluid communication with the buffer reservoir 33, and a dosing pump 31 attached the support base in fluid communication with the buffer reservoir 33. The dosing pump 31 is arranged for selective actuation by a user, and has a dispensing outlet 5 for expelling soap. An indicator rotor 15, which is associated with the housing is positioned outside of the soap content 35 and is coupled by a magnetic field to a float 41 within the buffer reservoir.

Also variants are conceivable in which the indicator rotor has multiple positions. For example, indicated by colors white, orange, red for content levels of respectively 100%, 30%, or less than 10%. This would be achieved by placing multiple magnets on top of each other in the indicator rotor each under a different angle. If the float with its float magnet moves along the indicator rotor, this will then always assume the relevant angular position.

It is thus believed that the operation and construction of the present invention will be apparent from the foregoing description and drawings appended thereto. Where in this specification or in the appended claims reference is made to "soap" this expression meant to encompass any liquid or viscous detergents, as well as any liquid or viscous disinfectants that are suitable for being dispensed from a dispensing device, as herein described. For the purpose of clarity and a concise description features are described herein as part of the same or separate embodiments, however, it will be appreciated that the scope of the invention may include embodiments having combinations of all or some of the features described. It will be clear to the skilled person that the invention is not limited to any embodiment herein described and that modifications are possible which may be considered within the scope of the appended claims. Also kinematic inversions are considered inherently disclosed and can be within the scope of the invention. In the claims, any reference signs shall not be construed as limiting the claim. The terms 'comprising' and 'including' when used in this description or the appended claims should not be construed in an exclusive or exhaustive sense but rather in an inclusive sense. Thus expression as 'including' or 'comprising' as used herein does not exclude the presence of other elements, additional structure or additional acts or steps in addition to those listed. Furthermore, the words 'a' and 'an' shall not be construed as limited to 'only one', but instead are used to mean 'at least one', and do not exclude a plurality. Features that are not specifically or explicitly described or claimed may additionally be included in the structure of the invention without departing from its scope. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. To the extend that structure, material, or acts are considered to be essential they are inexpressively indicated as such. Additions, deletions, and modifications within the purview of the skilled person may generally be made without departing from the scope of the invention, as determined by the claims.

## Claims

1. Soap dispenser, including:
a housing with a support base for mounting to a wall surface;
a cartridge containing a supply of soap removably supported on the support base, and having a liquid outlet;
a buffer reservoir having a liquid inlet for detachably receiving the liquid outlet of the cartridge for establishing fluid communication with the buffer reservoir; and
a dosing pump attached the support base in fluid communication with the buffer reservoir, and arranged for selective actuation by a user, the dosing pump further having a dispensing outlet for expelling soap,
wherein an indicator rotor associated with the housing is positioned outside of the soap content and is coupled by a magnetic field to a float within the buffer reservoir.

2. Soap dispenser as in claim 1, wherein the indicator rotor is journaled at its upper and lower axial ends for rotation about a substantially vertical axis, and accommodates an indicator permanent magnet.

3. Soap dispenser as in claim 2, wherein the float is mechanically guided for vertical translating movement, and carries a float permanent magnet.

4. Soap dispenser as in claim 3, wherein the float magnet is positioned on top of the float to be at any time positioned in an air space above an upper soap level in the buffer reservoir.

5. Soap dispenser as in claim 3 or 4, wherein the float magnet has its north pole positioned above its south pole aligned in a substantially vertical plane, and wherein the indicator magnet has its north and south poles aligned in a substantially horizontal plane.

6. Soap dispenser as in claim 4 or 5, wherein the float magnet with its permanent magnetic field interferes with the permanent magnetic field the indicator magnet of the indicator rotor.

7. Soap dispenser as in claim 6, wherein vertical movement of the float magnet changes polarity of the magnetic field to which the indicator magnet is exposed.

8. Soap dispenser as in claim 7, wherein polarity of the magnetic field is changed within a distance of 4 mm of float travel.

9. Soap dispenser as in one of claims 6, 7 or 8, wherein the indicator rotor rotates through 180° upon a change of the magnetic field.

10. Soap dispenser as in one of claims 2 to 9, wherein the indicator rotor is vertically divided into contrasting colored opposite halves.

11. Soap dispenser as in one of claims 1 to 10, further including a printed circuit board (PCB), and a local area network (LAN) module for transmitting data and/or receiving data within a network.

12. Soap dispenser according to claim 11, wherein the PCB includes a reed switch associated with the indicator rotor to open and close a contact under influence of the polarity of the magnetic field.

13. Soap dispenser according to claim 11 or 12, wherein the PCB includes sensors for recognizing a cartridge identity.

14. Network system for remotely providing service functionalities for a soap dispenser, comprising at least one cartridge based soap dispenser in accordance with one of claims 1 to 13, and a remote server in data communication with the at least one soap dispenser via a communication network, the at least one soap dispenser being adapted to monitor dispenser related parameters, and to submit the monitored parameters to the remote server, and wherein the remote server is adapted to initiate a service functionality depending on the submitted parameters.

15. Network system as in claim 14, wherein the at least one soap dispenser includes at least one sensor for monitoring dispenser related parameters, and a communication module for enabling communication with the remote server via the communication network.

16. Network system as in claim 15, wherein the at least one soap dispenser comprising a data storage for storing the monitored parameters, and wherein the communication module submits the stored parameters upon a request received from the remote server.

17. Network system as in claim 15 or 16, further comprising a control unit for starting a maintenance function if an indication of a corresponding service functionality is received by the communication module.

18. Network system as in one of claims 15, 16 or 17, wherein the at least one soap dispenser has a control module, wherein the communication module is connected to the control module, and wherein a communication program for communication to the server is being carried out by the control module of the soap dispenser or by a control module included in the communication module.

19. Network system as in one of claims 15 to 18, wherein the communication network comprises a dedicated network between the communication module and a public network.

20. Network system according to claim 19, wherein the dedicated network is a wireless network.

21. Network system as in one of claims 15 to 20, wherein the network includes a wireless data communication channel.
